# EUROPEAN PATENT APPLICATION

(11) **EP 4 717 187 A1**
(43) Date of publication of application: **01.04.2026**
(21) Application number: 25202925.1
(22) Date of filing: 18.09.2025
(51) Int. Cl.: A61B 8/12, A61B 1/00, A61B 1/005, A61M 25/01

(54) **OPERATION LEVER, MEDICAL DEVICE, ULTRASOUND PROBE, AND ENDOSCOPE**

(30) Priority: 30.09.2024 JP 2024170805
(71) Applicant: FUJIFILM Corporation, Tokyo 106-8620 (JP)
(72) Inventor: IWAKI, Takuro, Kanagawa, 258-8538 (JP)
(74) Representative: HGF

(57) **Abstract**

There are provided an operation lever, a medical device, an ultrasound probe, and an endoscope that allow an operator to easily perform an operation. An operation lever that is attached to an operation part for operating an insertion part to be inserted into a subject so as to be rotatable about a rotation axis, the operation lever includes an inner surface part that faces a side of the operation part, an outer surface part that faces a side opposite to the side of the operation part and has a width in a longitudinal direction of the operation part smaller than a width of the inner surface part, and an inclined surface part that is provided between the outer surface part and the inner surface part and is inclined with respect to the outer surface part.

## Description

### BACKGROUND OF THE INVENTION

### 1. Field of the Invention

The present invention relates to an operation lever, a medical device, an ultrasound probe, and an endoscope.

### 2. Description of the Related Art

For example, in the medical field, an endoscope inserted into a body of a subject such as a patient is known as one of medical devices. In general, the endoscope includes an insertion part having a bendable part and an operation part consecutively installed on a proximal end side of the insertion part. The operation part is provided with an operation lever.

For example, WO2015/166302A, JP2007-089966A, JP2005-160790A, and WO2012/147581A disclose an endoscope comprising two bending operation levers for an up-down direction and a left-right direction, which are pivotally supported on both side surfaces of an operation part. An operator grips the operation part and swings the bending operation lever in a longitudinal axis direction with a finger to bend the bendable part in four directions of up, down, left, and right remotely.

### SUMMARY OF THE INVENTION

By the way, in the endoscope comprising the above-described bending operation lever, the operator grips the operation part with an overhand grip or an underhand grip and performs a swinging operation on the bending operation lever with a finger. Therefore, it is desirable that the bending operation lever is easily swung regardless of the grip of the operator. However, the endoscopes of WO2015/166302A, JP2007-089966A, JP2005-160790A, and WO2012/147581A do not sufficiently consider the shape of the bending operation lever.

The present invention has been made in view of such circumstances, and aims to provide an operation lever, a medical device, an ultrasound probe, and an endoscope that are easy for an operator to operate.

In order to achieve the above object, an operation lever according to a first aspect of the present invention that is attached to an operation part for operating an insertion part to be inserted into a subject so as to be rotatable about a rotation axis, the operation lever comprises an inner surface part that faces a side of the operation part, an outer surface part that faces a side opposite to the side of the operation part and has a width in a longitudinal direction of the operation part smaller than a width of the inner surface part, and an inclined surface part that is provided between the outer surface part and the inner surface part and is inclined with respect to the outer surface part.

According to a second aspect of the present invention, in the operation lever according to the first aspect, the inclined surface parts are provided on both sides of the outer surface part in the longitudinal direction, respectively.

According to a third aspect of the present invention, in the operation lever according to the first aspect or second aspect, the inclined surface part is connected to the outer surface part.

According to a fourth aspect of the present invention, in the operation lever according to any one of the first to third aspects, the operation lever further comprises a finger hook part that is provided at a position away from the rotation axis and extends in a direction in which the rotation axis extends, and a shaft-like part that is provided between the rotation axis and the finger hook part and extends in a direction intersecting the rotation axis, in which the inclined surface part is provided on the finger hook part or the shaft-like part.

According to a fifth aspect of the present invention, in the operation lever according to the fourth aspect, the operation lever further comprises a bent part that is provided between the finger hook part and the shaft-like part.

According to a sixth aspect of the present invention, in the operation lever according to the fourth or fifth aspect, the shaft-like part has a side surface part having a normal component in the longitudinal direction, and the inclined surface part is provided on the side surface part.

According to a seventh aspect of the present invention, in the operation lever according to the sixth aspect, the side surface part includes a first region provided on a side of the rotation axis, a second region provided on a side of the finger hook part with respect to the first region and having a width in a direction of the rotation axis larger than a width of the first region, and a first change region provided between the first region and the second region and having a width in the direction of the rotation axis larger on a side of the second region than on a side of the first region, and the inclined surface part is provided on the second region or the first change region.

According to an eighth aspect of the present invention, in the operation lever according to any one of the fourth to seventh aspects, the outer surface part is provided on the shaft-like part, and the outer surface part of the shaft-like part includes a third region provided on a side of the rotation axis, a fourth region provided on a side of the finger hook part with respect to the third region and having a width in the longitudinal direction smaller than a width of the third region, and a second change region provided between the third region and the fourth region and having a width in the longitudinal direction smaller on a side of the fourth region than on a side of the third region.

According to a ninth aspect of the present invention, in the operation lever according to any one of the fourth to eighth aspects, the inclined surface part is provided on the finger hook part.

A medical device according to a tenth aspect including the insertion part and the operation part, comprises at least one operation lever according to any one of the first to ninth aspects.

According to an eleventh aspect of the present invention, in the medical device according to the tenth aspect, the at least one operation lever includes a first operation lever and a second operation lever, and the first operation lever and the second operation lever are disposed to face the operation part.

According to a twelfth aspect of the present invention, in the medical device according to any one of the first to eleventh aspects, the first operation lever and the second operation lever bend the insertion part in different directions.

An ultrasound probe according to a thirteenth aspect including the insertion part and the operation part and having a probe provided on a distal end side of the insertion part, comprises at least one operation lever according to any one of the first to ninth aspects.

An endoscope according to a fourteenth aspect including the insertion part and the operation part and having an observation window provided on a distal end side of the operation part, comprises at least one operation lever according to any one of the first to ninth aspects.

According to the present invention, operation is easier for an operator.

### BRIEF DESCRIPTION OF THE DRAWINGS

FIG. 1 is an overall perspective view of an ultrasound probe according to an embodiment.
FIG. 2 is a left side view of the ultrasound probe.
FIG. 3 is a right side view of the ultrasound probe.
FIG. 4 is a top view of the ultrasound probe.
FIG. 5 is a bottom view of the ultrasound probe.
FIG. 6 is a view of an operation lever as viewed from a Y(-) direction side.
FIG. 7 is a perspective view of the operation lever as viewed from an operation part side.
FIG. 8 is a perspective view of the operation lever as viewed from a side opposite to the operation part.
FIG. 9 is a view of the operation lever as viewed from an X(-) direction side.
FIG. 10 is a view of the operation lever as viewed from a Z(+) direction side.
FIG. 11 is a view of the operation lever as viewed from a Y(-) direction side.
FIG. 12 is a cross-sectional view of the operation lever.
FIG. 13 is an explanatory view showing an example of a case where an operator operates the operation lever.
FIG. 14 is an explanatory view showing an example of a case where the operator operates the operation lever.
FIG. 15 is an explanatory view showing an example of a case where the operator operates the operation lever.

### DESCRIPTION OF THE PREFERRED EMBODIMENTS

Hereinafter, a medical device according to an embodiment of the present invention will be described with reference to the accompanying drawings.

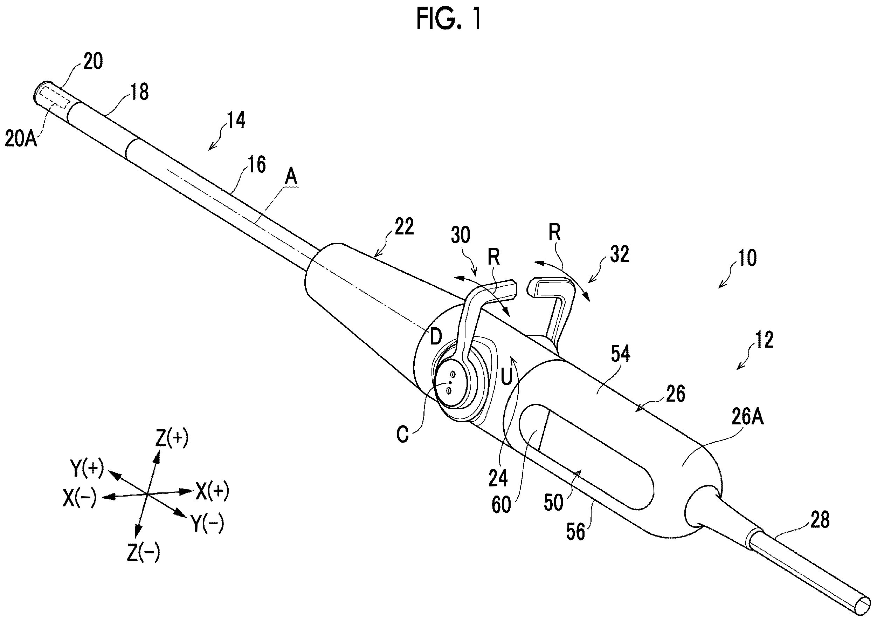

FIG. 1 is an overall perspective view of an ultrasound probe 10 according to an embodiment to which an operation lever according to the embodiment of the present invention is applied.

Hereinafter, in description of the configuration of each part of the ultrasound probe 10, an XYZ three-dimensional rectangular coordinate system will be used for the sake of convenience of description. In the drawings, a Z direction is a vertical direction, a Z(+) direction side is an upward direction, and a Z(-) direction is a downward direction. In addition, an X direction in the drawings is a lateral direction perpendicular to the Z direction, an X(+) direction side is a rightward direction, and an X(-) direction side is a leftward direction. In addition, in the drawings, a Y direction indicates a direction perpendicular to both the Z direction and the X direction, a Y(+) direction side indicates a distal end side direction, and a Y(-) direction side indicates a base end side direction. It should be noted that each of the above directions refers to a direction as viewed by the operator in a case where the operator grips the operation part 12 of the ultrasound probe 10 and looks at the ultrasound probe 10.

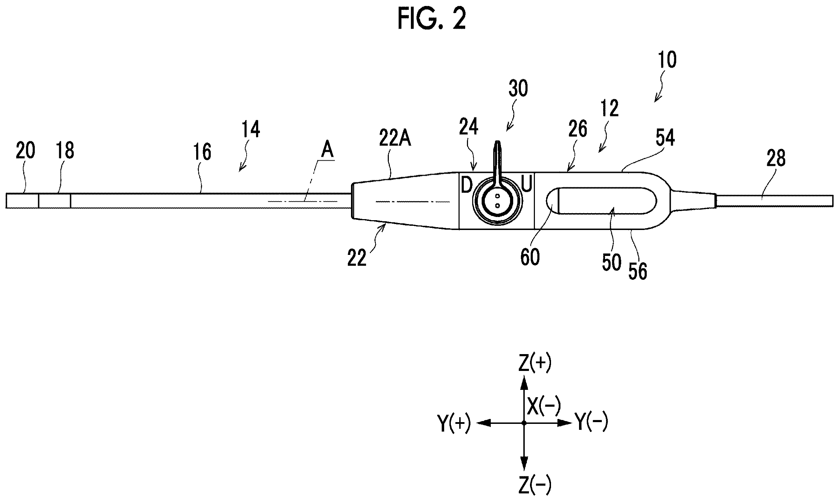

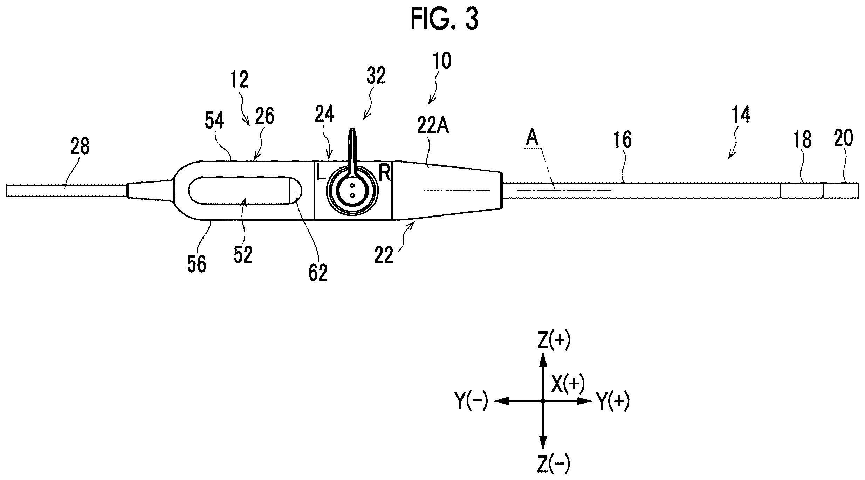

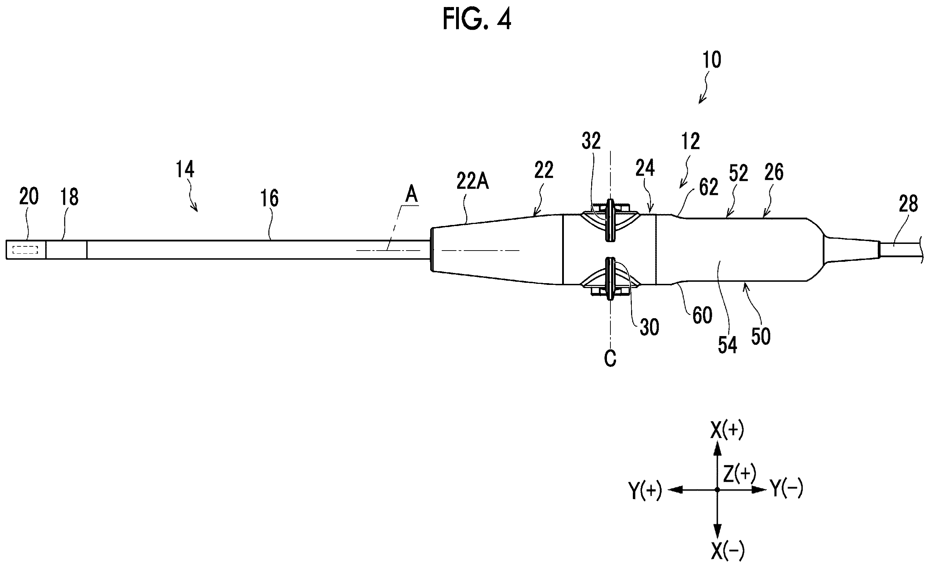

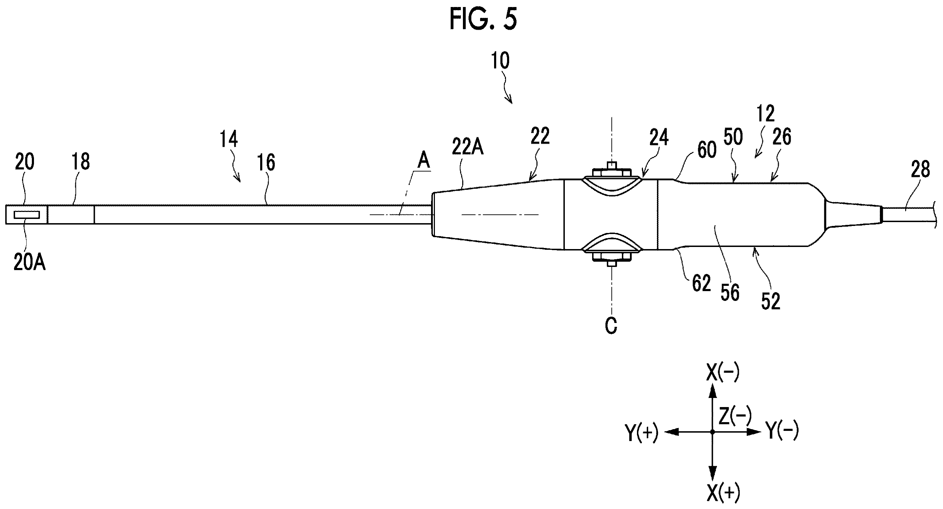

FIG. 2 is a left side view of the ultrasound probe 10 shown in FIG. 1 as viewed from the X(-) direction side. FIG. 3 is a right side view of the ultrasound probe 10 shown in FIG. 1 as viewed from the X(+) direction side. In addition, FIG. 4 is a top view of the ultrasound probe 10 shown in FIG. 1 as viewed from the Z(+) direction side. FIG. 5 is a bottom view of the ultrasound probe 10 shown in FIG. 1 as viewed from a Z(-) direction side.

As shown in FIGS. 1 to 5, the ultrasound probe 10 comprises the operation part 12 and an insertion part 14. The ultrasound probe 10 has a longitudinal axis A along a Y direction.

The insertion part 14 includes an insertion part main body 16, a bendable part 18 that is provided at a distal end side (Y(+) direction side) of the insertion part main body 16, and a probe 20 that is provided at a distal end side of a bendable part 18. The insertion part main body 16 of the present example is rigid. The operation part 12 and the insertion part 14 are examples of an operation part and an insertion part of the present invention.

The bendable part 18 is configured to be bendable by connecting, for example, a plurality of ring-shaped bending pieces along the longitudinal axis A, and is bent in an up-down direction (Z direction) and a left-right direction (X direction) by operations of an operation lever 30 and an operation lever 32 described below.

The probe 20 is a linear type in which an ultrasound wave emission surface 20A is formed to be flat. The probe 20 includes a plurality of oscillators that transmit and receive ultrasound waves to and from an imaging target portion (an organ, for example, a liver) of a subject. The probe 20 is connected to an ultrasound imaging apparatus (not shown) through a wiring line inserted into the insertion part 14 and the operation part 12. The probe 20 is not limited to the linear type probe, and may be a convex type or radial type probe.

The operation part 12 has a distal end part 22, an operation part main body 24, and a grip part 26 in order from a distal end side (Y(+) direction side) to a proximal end side (Y(-) direction side) in a longitudinal axis A direction. The longitudinal axis A direction is an example of a longitudinal direction of the present invention.

That is, the operation part 12 includes the operation part main body 24, the distal end part 22 provided on a distal end side of the operation part main body 24, and the grip part 26 provided on a proximal end side of the operation part main body 24. A cable 28 is connected to the proximal end portion of the grip part 26.

The distal end part 22 comprises a diameter-reduced part 22A of which an outer diameter is gradually reduced toward the insertion part 14. That is, the diameter-reduced part 22A has a tapered shape toward a Y(+) direction side. The distal end part 22 is not limited to the tapered shape.

The grip part 26 is configured in a cylindrical shape. In addition, in the grip part 26, the proximal end part 26A is formed in a hemispherical shape. The cable 28 is connected to the proximal end part 26A. The cable 28 encompasses a signal line connected to the probe 20.

The grip part 26 is a portion that is gripped by the right palm or the left palm of the operator. A holding surface 50 and a holding surface 52 are formed on the grip part 26. In addition, the holding surface 50 and the holding surface 52 are provided on sides facing each other with the longitudinal axis A interposed therebetween with respect to the grip part 26. That is, the holding surface 50 and the holding surface 52 are disposed at positions that are shifted from each other by 180 degrees in a circumferential direction of the grip part 26. The holding surface 50 is disposed on the X(-) direction side of the grip part 26, and the holding surface 52 is disposed on the X(+) direction side of the grip part 26.

The grip part 26 comprises an arc surface 54 and an arc surface 56 that connect the holding surface 50 and the holding surface 52. The arc surface 54 and the arc surface 56 are formed of arc-shaped curved surfaces, and distances from a central axis (not shown) of the grip part 26 along the longitudinal axis A are substantially equal. The arc surface 54 and the arc surface 56 are disposed at positions that are shifted from each other by 180 degrees in the circumferential direction of the grip part 26. The arc surface 54 is disposed on the Z(+) direction side of the grip part 26, and the arc surface 56 is disposed on the Z(-) direction side of the grip part 26.

The distances of the holding surfaces 50 and 52 from the central axis are shorter than the distances from the central axis to the arc surfaces 54 and 56 of the grip part 26. In this example, the holding surface 50 and the holding surface 52 are formed of surfaces. However, the holding surfaces 50 and 52 are not limited to flat surfaces and may be formed of convex surfaces or concave surfaces. For example, the holding surfaces 50 and 52 are formed as a flat surface, a convex surface (having a smaller curvature than the arc surface 54 and the arc surface 56), or a concave surface.

The holding surface 50 comprises an inclined surface 60 on the distal end side (Y(+) direction side). The inclined surface 60 is inclined toward a left side (X(-) direction side) as it goes toward the distal end side (Y(+) direction side). Similarly, the holding surface 52 comprises an inclined surface 62 on the distal end side (Y(+) direction side). The inclined surface 62 is inclined toward a right side (X(+) direction side) as it goes the distal end side (Y(+) direction side). The distal end sides of the inclined surface 60 and the inclined surface 62 are formed in an arc shape toward the distal end side (Y(+) direction side).

In a case where the operator grips the grip part 26, one surface of the holding surface 50 and the holding surface 52 is gripped by the palm, and the other surface is gripped by the middle finger, the ring finger, and the little finger, making it easy to grip. The holding surface 50 and the holding surface 52 are connected by the arc surface 54 and the arc surface 56 to form a ridge line along the longitudinal axis A at each connection point. The ridge line makes it easier for the fingers to catch, thereby facilitating twisting of the ultrasound probe 10.

The operation part main body 24 is configured in a substantially cylindrical shape. The operation lever 30 and the operation lever 32 are rotatably provided on an outer surface of the operation part main body 24. The operation lever 30 and the operation lever 32 are operation members for bending the bendable part 18. The operation lever 30 and the operation lever 32 are examples of an operation lever according to the embodiment of the present invention. The operation lever 30 and the operation lever 32 are also examples of a first operation lever and a second operation lever of the present invention.

The operation lever 30 and the operation lever 32 are symmetrically disposed on opposite sides facing each other with the operation part main body 24 interposed therebetween. That is, the operation lever 30 and the operation lever 32 are disposed at positions that are shifted from each other by 180 degrees in the circumferential direction of the operation part main body 24. The operation lever 30 and the operation lever 32 are disposed side by side along the X direction to face each other. The operation lever 30 and the operation lever 32 are rotatably attached to the operation part main body 24, which constitutes a part of the operation part 12, about a rotation axis C. Although the shapes will be described later, the operation lever 30 and the operation lever 32 have the same shape and have a substantially L-shape.

The operation lever 30 and the operation lever 32 are in an upright state along the Z-axis direction in a non-operated position (neutral position). The non-operated position is a position at which the operator does not apply a force to the operation lever 30 and the operation lever 32. The operation lever 30 and the operation lever 32 can be rotated by the operator in a rotation direction R (Y(+) direction side and Y(-) direction side, that is, front-rear direction) with respect to the non-operated position in a specific angular range about the rotation axis C. The operation lever 30 and the operation lever 32 can be rotated independently of each other. Structures of the operation lever 30 and the operation lever 32 will be described later. In this example, a direction of the rotation axis C is parallel to the X direction.

As shown in FIGS. 1 and 2, characters "D" and "U" are shown on the outer surface of the operation part main body 24 as viewed from the left side surface side. In this example, "D" is displayed on the distal end side, and "U" is displayed on the proximal end side. In addition, as shown in FIG. 3, characters "R" and "L" are shown on the outer surface of the operation part main body 24 as viewed from the right side surface side. "R" is displayed on the distal end side, and "L" is displayed on the proximal end side. "D", "U", "R", and "L" indicate "Down", "Up", "Right", and "Left" which are bending directions of the bendable part 18. The operator can easily understand a relationship between the operation directions of the operation lever 30 and the operation lever 32 and the bending direction of the bendable part 18.

### Overall Shape of Operation Lever

Next, the shapes of the operation levers 30 and 32 of the embodiment will be described. Incidentally, since the operation lever 30 and the operation lever 32 have the same shape, the operation lever 30 will be described.

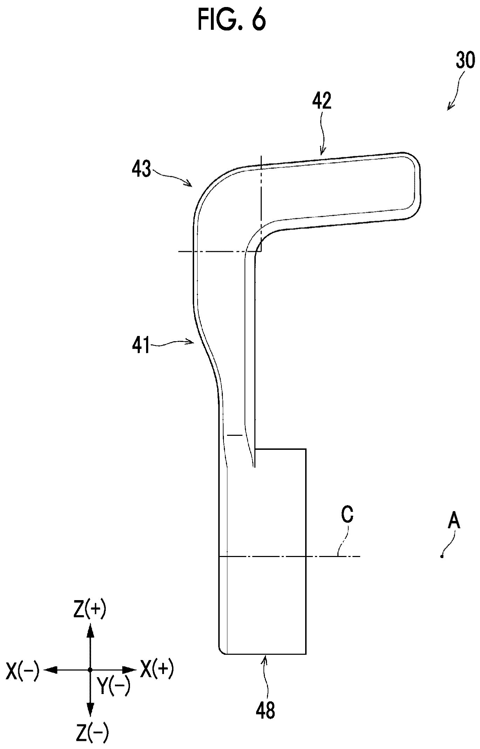

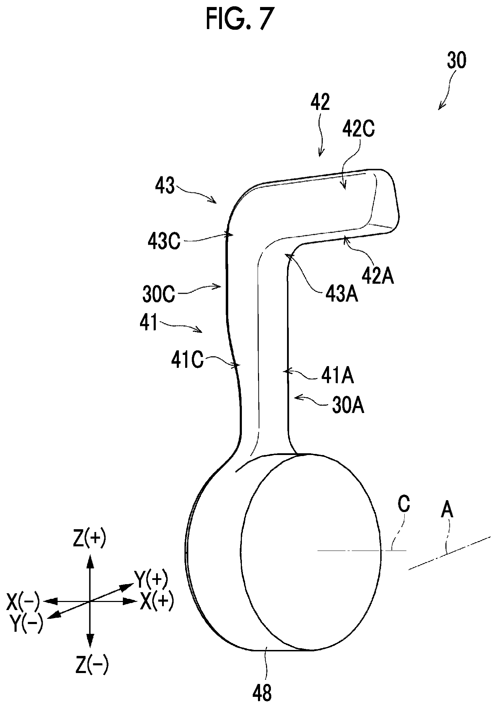

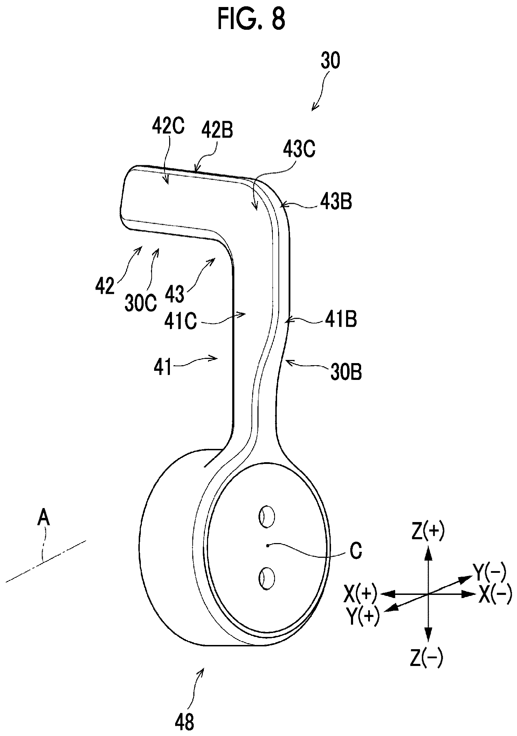

FIG. 6 is a front view of the operation lever 30 as viewed from the Y(-) direction side. FIG. 7 is a perspective view of the operation lever 30 as viewed from the Y(-) direction side, and FIG. 8 is a perspective view of the operation lever 30 as viewed from the Y(+) direction side. The operation lever 30 is shown at the non-operated position in any of FIGS. 6 to 8. Hereinafter, in a case of describing the configuration of each part of the operation lever 30, a case where the operation lever 30 is in a non-operated position will be described as a premise.

The operation lever 30 has a substantially L-shaped shape, and has a shaft-like part 41, a finger hook part 42, and a bent part 43. The operation lever 30 has a disk part 48 on the base side (the rotation axis C side) of the shaft-like part 41.

The shaft-like part 41 extends toward the Z(+) direction side (that is, along a direction orthogonal to the rotation axis C) with the disk part 48 as a starting point. In the present example, a case where the direction in which the shaft-like part 41 extends is the direction orthogonal to the rotation axis C has been described, but the present invention is not limited to this, and the shaft-like part 41 may be provided to extend in a direction oblique to the rotation axis C. That is, the direction in which the shaft-like part 41 extends may be a direction intersecting the rotation axis C.

The finger hook part 42 is connected to the disk part 48 via the shaft-like part 41 and the bent part 43, and is provided along a direction slightly inclined with respect to the rotation axis C. That is, the finger hook part 42 is provided at a position away from the disk part 48 (that is, a position away from the rotation axis C) and extends along a direction substantially parallel to the rotation axis C. Specifically, the finger hook part 42 is provided such that a distance from the rotation axis C gradually increases toward the inside (X(+) direction side) of the operation part 12 (operation part main body 24). The finger hook part 42 only needs to extend at least in a direction intersecting the rotation axis C, may be parallel to the rotation axis C, or may have a larger inclination angle with respect to the rotation axis C than that in the present example.

The bent part 43 is provided between the shaft-like part 41 and the finger hook part 42, and is a portion that connects end parts of the shaft-like part 41 and the finger hook part 42 to each other. The bent part 43 may have any shape as long as the end parts of the shaft-like part 41 and the finger hook part 42 can be connected to each other, and may smoothly connect the shaft-like part 41 and the finger hook part 42 in an R shape (curved shape) as in the present example, or may connect the shaft-like part 41 and the finger hook part 42 at a right angle or an acute angle, which is not shown.

The disk part 48 is a portion that is attached to the operation part main body 24 (not shown) in the operation lever 30. In a case where the disk part 48 is attached to the operation part main body 24, the operation lever 30 can be rotated about the rotation axis C.

As shown in FIGS. 7 and 8, the operation lever 30 includes an inner surface part 30A and an outer surface part 30B. The inner surface part 30A is a surface of the operation lever 30 that faces a side on which the operation part 12 is located in a state in which the operation lever 30 is attached to the operation part 12 (operation part main body 24). The inner surface part 30A has a shape corresponding to each part of the operation lever 30, and specifically, has a first inner surface part 41A corresponding to the shaft-like part 41, a second inner surface part 42A corresponding to the finger hook part 42, and a third inner surface part 43A corresponding to the bent part 43. The inner surface part 30A is an example of an inner surface part of the present invention.

The outer surface part 30B is a surface of the operation lever 30 that faces a side opposite to a side on which the operation part 12 is located in a state in which the operation lever 30 is attached to the operation part 12 (operation part main body 24). The outer surface part 30B has a shape corresponding to each part of the operation lever 30, and specifically, has a first outer surface part 41B corresponding to the shaft-like part 41, a second outer surface part 42B corresponding to the finger hook part 42, and a third outer surface part 43B corresponding to the bent part 43. The outer surface part 30B is an example of an outer surface part of the present invention.

Further, the operation lever 30 has two side surface parts 30C, respectively facing the front-rear direction (that is, the Y(+) direction side and the Y(-) direction side). That is, the two side surface parts 30C are provided between the inner surface part 30A and the outer surface part 30B, and each of the two side surface parts 30C has a normal component in the longitudinal axis A direction. Each of the two side surface parts 30C has an L-shape, and specifically, has a first side surface part 41C corresponding to the shaft-like part 41, a second side surface part 42C corresponding to the finger hook part 42, and a third side surface part 43C corresponding to the bent part 43. Each part of the side surface part 30C is provided with an inclined surface part, which will be described later, in order to facilitate the rotational operation of the operation levers 30 and 32.

### Configuration of Shaft-Like Part

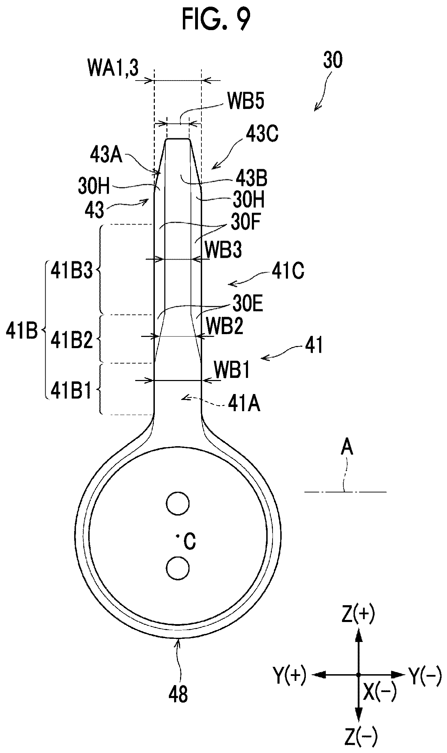

The configuration of the shaft-like part 41 will be described in detail. FIG. 9 is a view of the operation lever 30 as viewed from an X(-) direction side. As shown in FIG. 9, the first outer surface part 41B of the shaft-like part 41 has a first off-axis region 41B1, a second off-axis region 41B2, and a third off-axis region 41B3 in order from the rotation axis C side toward the finger hook part 42 side. A relationship in a width direction between the first off-axis region 41B1, the second off-axis region 41B2, and the third off-axis region 41B3 is as follows. In the following description, the width direction of each region constituting the shaft-like part 41 is the longitudinal axis A direction (that is, a Y-axis direction).

A width WB1 of the first off-axis region 41B1 is constant from the rotation axis C side toward the finger hook part 42 side. On the other hand, a width WB3 of the third off-axis region 41B3 is constant from the rotation axis C side toward the finger hook part 42 side, and the width WB3 is smaller than the width WB1 of the first off-axis region 41B1 (WB3 < WB1).

The second off-axis region 41B2 is a region that connects the first off-axis region 41B1 and the third off-axis region 41B3, and a width WB2 of the second off-axis region 41B2 gradually decreases from the first off-axis region 41B1 side toward the third off-axis region 41B3 side. Specifically, the second off-axis region 41B2 is formed in a tapered shape consisting of a straight line having a narrow width toward the Z(+) direction side. The width WB2 thereof is equal to the maximum width on the side of the first off-axis region 41B1, which is equal to the width WB1 of the first off-axis region 41B1, and is equal to the minimum width on the side of the third off-axis region 41B3, which is equal to the width WB3 of the third off-axis region 41B3, and the width changes from the maximum width to the minimum width toward the Z(+) direction. The second off-axis region 41B2 is not limited to the tapered shape consisting of a straight line as in the present example as long as the width WB2 is smaller on the side of the third off-axis region 41B3 than on the side of the first off-axis region 41B1, and may be, for example, a tapered shape consisting of a curved line, or may include a region having a constant width in a part of the second off-axis region 41B2. The first off-axis region 41B1, the second off-axis region 41B2, and the third off-axis region 41B3 are examples of a third region, a second change region, and a fourth region of the present invention, respectively.

In the present example, in the operation lever 30, a width WA1 of the first inner surface part 41A corresponding to the shaft-like part 41 is constant from the rotation axis C side toward the finger hook part 42 side, and is equal to the width WB1 of the first off-axis region 41B1 of the first outer surface part 41B corresponding to the shaft-like part 41 (WA1 = WB1). The width WA1 of the first inner surface part 41A may be larger than the width WB1 of the first off-axis region 41B1.

Since the first off-axis region 41B1, the second off-axis region 41B2, and the third off-axis region 41B3 have the above-described relationship in the width direction, the width WB3 of the third off-axis region 41B3 is smaller than the width WA1 of the first inner surface part 41A (WB3 < WA1). In addition, the width WB2 of the second off-axis region 41B2 changes from the minimum width to the maximum width as described above, but the width WB2 is at least equal to or less than the width WA1 of the first inner surface part 41A (WB2 ≤ WA1).

In this way, by making the width of the outer surface (first outer surface part 41B) and the width of the inner surface (first inner surface part 41A) of the operation lever 30 different from each other, the first side surface part 41C corresponding to the shaft-like part 41 among the two side surface parts 30C connecting the outer surface and the inner surface can be configured as the inclined surface part.

Specifically, a surface part connected to the second off-axis region 41B2 among the two first side surface parts 41C is configured as a first inclined surface part 30E. The first inclined surface part 30E is formed of a planar surface that is obliquely inclined with respect to the outer surface part 30B (second off-axis region 41B2). In other words, two first inclined surface parts 30E are connected to the second off-axis region 41B2, and the two first inclined surface parts 30E are configured to spread in the Y-axis direction from the outer surface part 30B toward the inner surface part 30A in the X(+) direction side, based on the outer surface part 30B (second off-axis region 41B2). Since the width WB2 of the second off-axis region 41B2 of the first inclined surface part 30E changes from the minimum width to the maximum width as described above, the first inclined surface part 30E has an inclination corresponding to the change in the width WB2 of the second off-axis region 41B2.

In addition, a surface part connected to the third off-axis region 41B3 among the two first side surface parts 41C is configured as a second inclined surface part 30F. The second inclined surface part 30F is formed of a planar surface that is obliquely inclined with respect to the outer surface part 30B (third off-axis region 41B3). In other words, two second inclined surface parts 30F are connected to the third off-axis region 41B3, and the two second inclined surface parts 30F are configured to spread in the Y-axis direction from the outer surface part 30B toward the inner surface part 30A in the X(+) direction side, based on the outer surface part 30B (third off-axis region 41B3).

In the present embodiment, the configuration in which the inclined surface part is provided on each of the two first side surface parts 41C (that is, the configuration in which the inclined surface part is provided on both side surface parts of the operation lever 30) has been described, but the present invention is not limited to this configuration. For example, a configuration in which the inclined surface part is provided on only one side surface part of both side surface parts of the operation lever 30 may be adopted. The same applies to a third inclined surface part 30G and a fourth inclined surface part 30H, which will be described later.

In addition, in the present embodiment, the configuration in which the first inclined surface part 30E and the second inclined surface part 30F are provided on the first side surface part 41C has been described, but the present invention is not limited to this, and only one of the first inclined surface part 30E or the second inclined surface part 30F may be provided.

In addition, in the present embodiment, the case where the first inclined surface part 30E and the second inclined surface part 30F are formed of a planar surface inclined obliquely with respect to the outer surface part 30B has been described, but the present invention is not limited to this. For example, the first inclined surface part 30E and the second inclined surface part 30F may be formed of a curved surface inclined obliquely with respect to the outer surface part 30B. The same applies to a third inclined surface part 30G and a fourth inclined surface part 30H, which will be described later.

### Configuration of Finger Hook Part

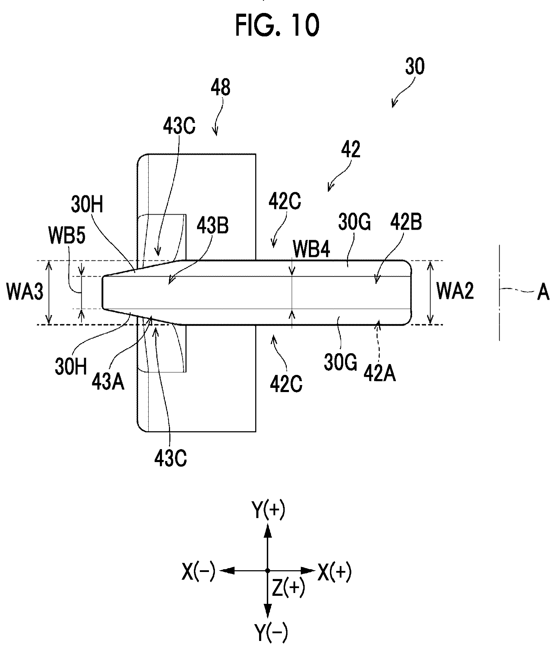

Next, a configuration of the finger hook part 42 will be described in detail. FIG. 10 is a view of the operation lever 30 as viewed from a Z(+) direction side. In the following description, a width direction of each part of the finger hook part 42 is the longitudinal axis A direction (that is, the Y-axis direction).

As shown in FIG. 10, in a case where the operation lever 30 is viewed from the Z(+) direction side, the second outer surface part 42B of the finger hook part 42 extends along an X-axis direction. A width WB4 of the second outer surface part 42B is a constant width along the X-axis direction which is an extension direction thereof. The width WB4 is equal to the width WB3 (see FIG. 9) of the third off-axis region 41B3 described above.

On the other hand, the second inner surface part 42A of the finger hook part 42 has the same point as the above-described second outer surface part 42B in that the second inner surface part 42A has a constant width along the X-axis direction which is the extension direction, but the width WA2 of the second inner surface part 42A is configured to be larger than the width WB4 of the second outer surface part 42B (WA2 > WB4).

In this way, by making the width of the outer surface (second outer surface part 42B) and the width of the inner surface (second inner surface part 42A) of the operation lever 30 different from each other, the second side surface part 42C corresponding to the finger hook part 42 among the two side surface parts 30C connecting the outer surface and the inner surface can be configured as the inclined surface part.

Specifically, the two second side surface parts 42C are each a surface part connected to the outer surface part 30B (second outer surface part 42B), and are configured as the third inclined surface part 30G. The third inclined surface part 30G is formed of a planar surface that is obliquely inclined with respect to the outer surface part 30B (second outer surface part 42B). In other words, two third inclined surface parts 30G are connected to the second outer surface part 42B, and the two third inclined surface parts 30G are configured to spread in the Y-axis direction from the outer surface part 30B toward the inner surface part 30A in the Z(-) direction side based the outer surface part 30B (second outer surface part 42B).

In the present embodiment, the configuration in which the inclined surface part is provided on each of the two second side surface parts 42C (that is, the configuration in which the inclined surface part is provided on both side surface parts of the operation lever 30) has been described, but the present invention is not limited to this configuration. For example, a configuration in which the inclined surface part is provided on only one side surface part of both side surface parts of the operation lever 30 may be adopted.

### Configuration of Bent Part

Next, a configuration of the bent part 43 will be described in detail with reference to FIGS. 9 and 10. In the following description, a width direction of each part of the bent part 43 is the longitudinal axis A direction (that is, the Y-axis direction).

As shown in FIG. 9, in a case where the operation lever 30 is viewed from the X(-) direction side, the third outer surface part 43B in the bent part 43 extends along a Z-axis direction. As shown in FIG. 10, in a case where the operation lever 30 is viewed from the Z(+) direction side, the third outer surface part 43B of the bent part 43 extends along the X-axis direction. A width WB5 of the third outer surface part 43B is a constant width along the X-axis direction from the Z-axis direction, which is an extension direction thereof. The width WB5 is equal to the width WB3 of the third off-axis region 41B3 and the width WB4 of the second outer surface part 42B.

On the other hand, the width WA3 of the third inner surface part 43A in the bent part 43 has the same point as the width WB5 in that the width WA3 is constant along the extending direction, but the width WA3 of the third inner surface part 43A is configured to be larger than the width WB5 of the third outer surface part 43B (WA3 > WB5).

By making the width of the outer surface (third outer surface part 43B) and the width of the inner surface (third inner surface part 43A) of the operation lever 30 different from each other, the third side surface part 43C corresponding to the bent part 43 among the two side surface parts 30C connecting the outer surface and the inner surface can be configured as the inclined surface part.

Specifically, the two third side surface parts 43C are each a surface part connected to the outer surface part 30B (third outer surface part 43B), and are configured as the fourth inclined surface part 30H. The fourth inclined surface part 30H is formed of a planar surface that is obliquely inclined with respect to the outer surface part 30B (third outer surface part 43B). In other words, two fourth inclined surface parts 30H are connected to the third outer surface part 43B, and the two fourth inclined surface parts 30H are configured to spread in the Y-axis direction from the outer surface part 30B toward the inner surface part 30A in the X(-) direction side and the Z(-) direction side based on the outer surface part 30B (third outer surface part 43B).

### Shape of Side Surface Part of Shaft-Like Part

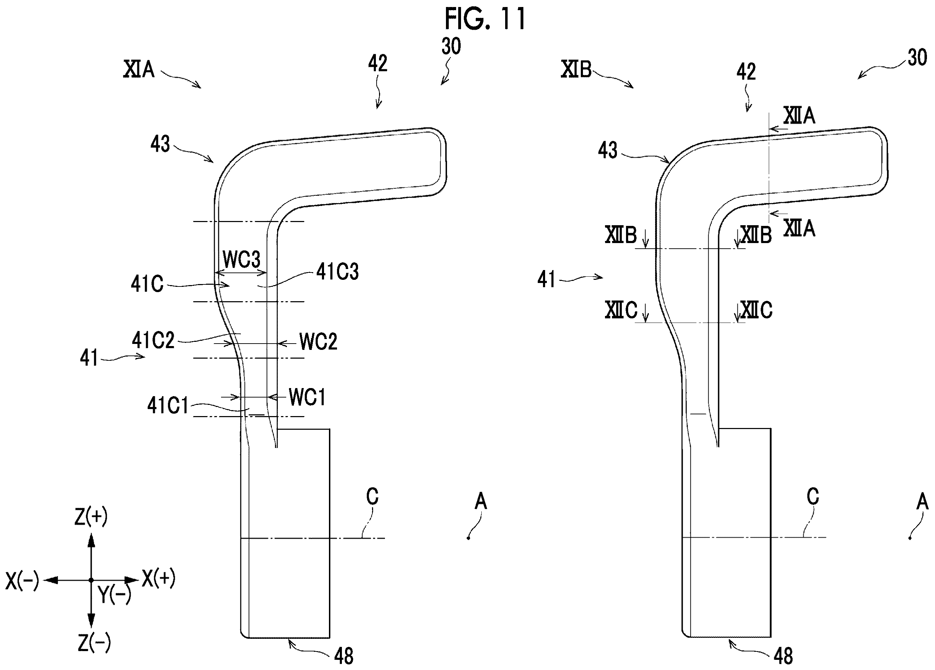

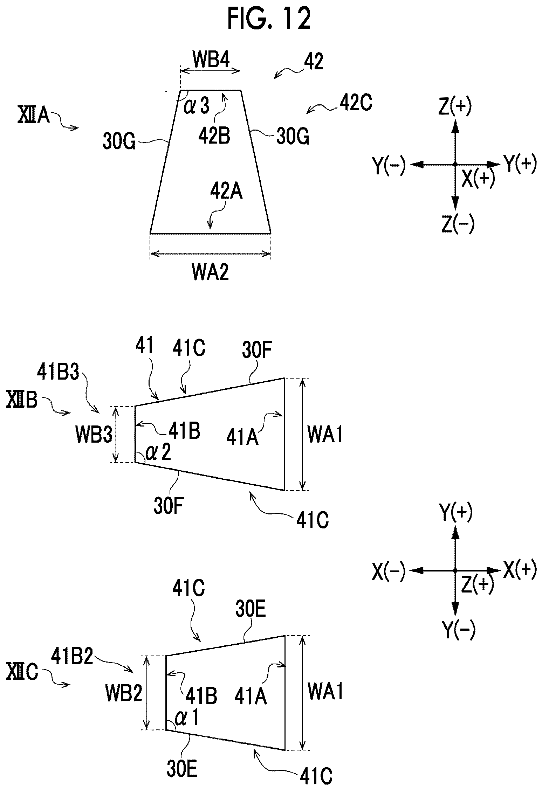

Next, a shape of the first side surface part 41C of the shaft-like part 41 will be described in more detail with reference to FIG. 11. XIA of FIG. 11 is a view of the operation lever 30 as viewed from the Y(-) direction side, similarly to FIG. 6. XIB of FIG. 11 is a view showing a position of a cross-sectional view shown in FIG. 12. In the description regarding the shape of the first side surface part 41C of the shaft-like part 41, a width direction of the first side surface part 41C is a direction of the rotation axis C (X-axis direction).

As shown in XIA of FIG. 11, the first side surface part 41C of the shaft-like part 41 is formed to be wider on the finger hook part 42 side than on the rotation axis C side, and specifically, the first side surface part 41C has a first axis side region 41C1, a second axis side region 41C2, and a third axis side region 41C3 in this order from the rotation axis C side toward the finger hook part 42 side. Although described below, since the first side surface part 41C has a wide portion, a contact area between the operator's finger and the operation levers 30 and 32 can be increased.

The first axis side region 41C1, the second axis side region 41C2, and the third axis side region 41C3 each have a width WC1, a width WC2, and a width WC3 in a rotation axis C direction. A relationship between the first axis side region 41C1, the second axis side region 41C2, and the third axis side region 41C3 in the width direction is as follows. In the following description, a width direction of each region constituting the shaft-like part 41 is the rotation axis C direction (that is, an X-axis direction).

The width WC1 of the first axis side region 41C1 is a constant width from the rotation axis C side toward the finger hook part 42 side. The width WC3 of the third axis side region 41C3 is a constant width from the rotation axis C side toward the finger hook part 42 side, and is larger than the width WC1 of the first axis side region 41C1 (WC3 > WC1).

The second axis side region 41C2 is a region that connects the first axis side region 41C1 and the third axis side region 41C3, and the width WC2 thereof gradually increases from the first axis side region 41C1 side toward the third axis side region 41C3 side. Specifically, the second axis side region 41C2 is formed in a tapered shape consisting of a straight line having a wide width toward the Z(+) direction side. The width WC2 is equal to the minimum width on the side of the first axis side region 41C1, which is equal to the width WC1 of the first axis side region 41C1, and is equal to the maximum wider on the third axis side region 41C3, which is equal to the width WC3 of the third axis side region 41C3, and the width changes from the minimum width to the maximum width toward the Z(+) direction side. The second axis side region 41C2 is not limited to the tapered shape consisting of a straight line as in the present example as long as the width WC2 is larger on the side of the third axis side region 41C3 than on the side of the first axis side region 41C1, and may be, for example, a tapered shape consisting of a curved line, or may include a region having a constant width in a part of the second axis side region 41C2. The first axis side region 41C1, the second axis side region 41C2, and the third axis side region 41C3 are examples of a first region, a first change region, and a second region of the present invention, respectively.

In the present embodiment, in each region (the first axis side region 41C1, the second axis side region 41C2, and the third axis side region 41C3) constituting the shaft-like part 41, an inner edge part corresponding to the inner side of the operation part 12 (operation part main body 24) is formed in a linear shape parallel to the Z-axis direction, and an outer edge part corresponding to the outer side of the operation part 12 (operation part main body 24) on the opposite side is formed in a linear shape parallel or obliquely inclined to the Z-axis direction according to the width of each region. However, the present invention is not limited thereto. For example, not only the outer edge part but also the inner edge part may have the same configuration as the outer edge part as long as the above-described relationship in the width direction is satisfied. Furthermore, the outer edge part or the inner edge part may be formed in a curved shape parallel or obliquely inclined to the Z-axis direction.

### Cross-Sectional Shape of Shaft-Like Part and Finger Hook Part

Next, the cross-sectional shapes of the shaft-like part 41 and the finger hook part 42 will be described with reference to FIG. 12. XIIA of FIG. 12 is a cross-sectional view of the finger hook part 42 taken along a line XIIA-XIIA of XIB of FIG. 11. XIIB of FIG. 12 is a cross-sectional view of the third axis side region 41C3 along a line XIIB -XIIB of XIB of FIG. 11, and XIIC of FIG. 12 is a cross-sectional view of the second axis side region 41C2 along a line XIIC -XIIC of XIB of FIG. 11.

As shown in XIIA of FIG. 12, in the finger hook part 42, the third inclined surface part 30G is connected to the second outer surface part 42B and the second inner surface part 42A, and spreads to the Y(+) direction side and the Y(-) direction side from the second outer surface part 42B toward the second inner surface part 42A. An inclined angle α3 of the third inclined surface part 30G is an angle formed by the second outer surface part 42B and the third inclined surface part 30G, and the angle is an obtuse angle (α3 > 90°).

As shown in XIIB of FIG. 12, in the shaft-like part 41, the second inclined surface part 30F is connected to the first outer surface part 41B and the first inner surface part 41A, and spreads to the Y(+) direction side and the Y(-) direction side from the first outer surface part 41B toward the first inner surface part 41A. An inclined angle α2 of the second inclined surface part 30F is an angle formed by the first outer surface part 41B and the second inclined surface part 30F, and the angle is an obtuse angle (α2 > 90°).

As shown in XIIC of FIG. 12, in the shaft-like part 41, the first inclined surface part 30E is connected to the first outer surface part 41B and the first inner surface part 41A, and spreads to the Y(+) direction side and the Y(-) direction side from the first outer surface part 41B toward the first inner surface part 41A. An inclined angle α1 of the first inclined surface part 30E is an angle formed by the first outer surface part 41B and the first inclined surface part 30E, and the angle is an obtuse angle (α1 > 90°). It should be noted that the inclined angle α1 of the first inclined surface part 30E and the inclined angle α2 of the second inclined surface part 30F are different from each other.

### Use Example of Operation Lever

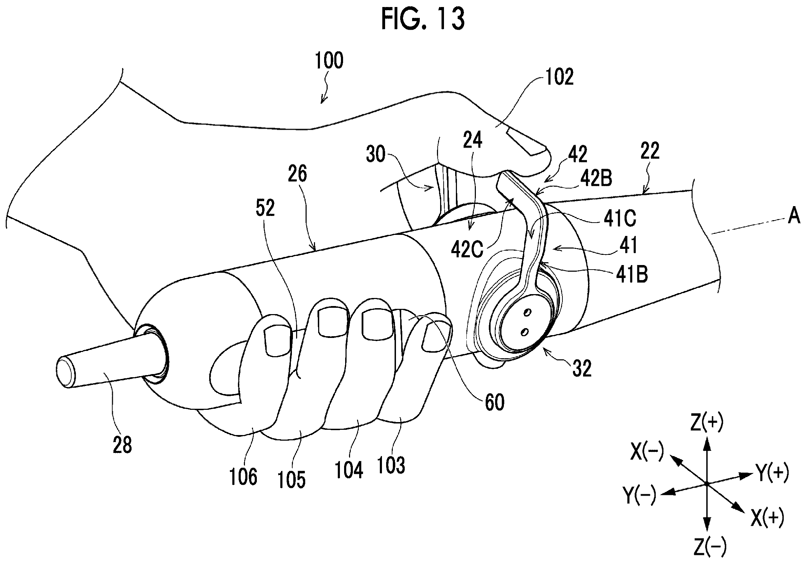

Next, an example of a use example of the operation levers 30 and 32 will be described with reference to FIGS. 13 to 15.

FIG. 13 shows a state immediately before the operation lever 32 is operated. First, the operator grips the grip part 26 with a left hand 100. Specifically, the operator holds the holding surface 50 (not shown) of the two holding surfaces 50 and 52 of the grip part 26 with the palm, and holds the holding surface 52 with an index finger 103, a middle finger 104, a ring finger 105, and a little finger 106. The operator presses a thumb 102 against the finger hook part 42 of the operation lever 32.

As described above, the finger hook part 42 is configured such that the width of the second outer surface part 42B in the longitudinal axis A direction is narrower than the width of the second inner surface part 42A (not shown). In a case where the force is applied, the finger hook part 42 is likely to bite into the thumb 102, and the thumb 102 is unlikely to slip on the finger hook part 42. In addition, the thumb 102 can easily reach the two second side surface parts 42C. Since the third inclined surface part 30G (not shown) is provided on the second side surface part 42C of the finger hook part 42 as described above, the thumb 102 is easily placed on the finger hook part 42. Therefore, it is easy for the operator to operate the operation lever 32 back and forth (operation in the rotation direction R).

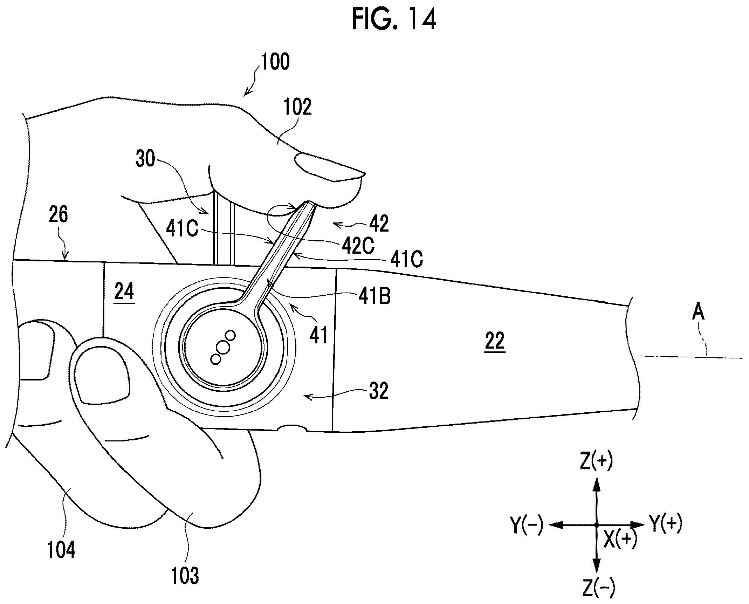

FIG. 14 shows a state in which the operator rotates the operation lever 32 in a forward direction. In the operation lever 32 of the present example, the third inclined surface part 30G (not shown) is provided on each of the two second side surface parts 42C.

In a case where the operation lever 32 is rotated in the forward direction, the operator can press the second side surface part 42C of the finger hook part 42 with the entire thumb 102 by the third inclined surface part 30G on the Y(-) direction side, and thus the operator can rotate the operation lever 32 with a light force.

In addition, in a case where the operator rotates the operation lever 32 in a rearward direction, the operator can pull the second side surface part 42C of the finger hook part 42 with the entire thumb 102 by the third inclined surface part 30G on the Y(+) direction side, and thus the operator can rotate the operation lever 32 with a light force.

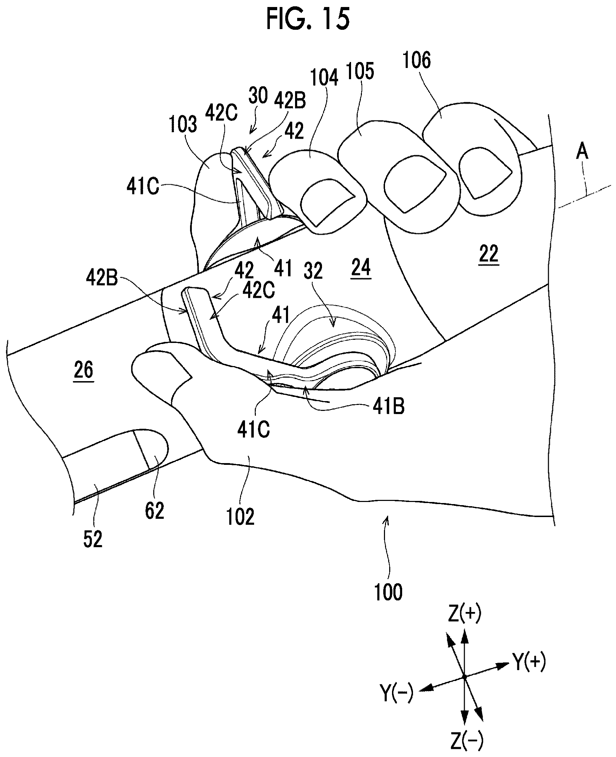

FIG. 15 shows a gripping state different from those in FIGS. 13 and 14. As shown in FIG. 15, the operator grips the distal end part 22 with the left hand 100. Specifically, the operator holds the distal end part 22 with the palm, the middle finger 104, the ring finger 105, and the little finger 106. The thumb 102 touches the operation lever 32, and the index finger 103 touches the operation lever 30. The shaft-like parts 41 of the operation levers 30 and 32 have the second axis side region 41C2 and the third axis side region 41C3 (not shown) on the first side surface part 41C. By having these regions, a contact area between the thumb 102 and the operation lever 32 is increased, and the operator can accurately and firmly operate the operation lever 32. Similarly, by having these regions, the contact area between the index finger 103 and the operation lever 30 is increased, and the operator can accurately and firmly operate the operation lever 30.

In addition, the shaft-like parts 41 of the operation levers 30 and 32 have the first inclined surface part and the second inclined surface part (not shown) on the first side surface part 41C. By having these inclined surface parts, the thumb 102 is likely to bite into the first outer surface part 41B of the operation lever 32, and the thumb 102 is likely to be placed on the first side surface part 41C. Similarly, by having these inclined surface parts, the index finger 103 is likely to bite into the first outer surface part 41B of the operation lever 30, and the index finger 103 is likely to be placed on the first side surface part 41C.

As described above, since the operation levers 30 and 32 of the present example have the inclined surface part inclined with respect to the outer surface part 30B in the side surface part 30C provided between the outer surface part 30B and the inner surface part 30A, the operator can easily perform the rotation operation.

### Other Forms of Operation Lever

In the embodiment, the case where the inclined surface part is provided on the entire first side surface part 41C of the shaft-like part 41, the second side surface part 42C of the finger hook part 42, and the third side surface part 43C of the bent part 43 has been exemplified, but the inclined surface part can be provided only on a part of the side surface part 30C of each part of the operation lever 30. For example, the inclined surface part may be formed only on the outer surface part 30B side in the side surface part 30C. Specifically, the second inclined surface part 30F may be formed only on the first outer surface part 41B side in the first side surface part 41C. The first inclined surface part 30E may be formed only on the first outer surface part 41B side in the first side surface part 41C. In addition, the third inclined surface part 30G may be formed only on the second outer surface part 42B side in the second side surface part 42C. The third inclined surface part 30G may be formed only on a side of the second side surface part 42C opposite to the bent part 43 side.

In the embodiment, the operation levers 30 and 32 are formed in a substantially L-shape by the shaft-like part 41, the bent part 43, and the finger hook part 42, but the present invention is not limited thereto. For example, the operation levers 30 and 32 may be formed in a substantially I-shape that does not have the bent part 43, in which the shaft-like part 41 and the finger hook part 42 are directly connected to each other and extend linearly along the Z-axis direction.

Specifically, the operation lever having a substantially I-shape is the same as the operation lever 30 of the embodiment in that the operation lever has the shaft-like part 41 shown in FIGS. 9 and 11 and the finger hook part 42 shown in FIG. 10, but the shaft-like part 41 and the finger hook part 42 are directly connected to each other in the operation lever having a substantially I-shape. The second inner surface part 42A, the second outer surface part 42B, and the second side surface part 42C of the finger hook part 42 are connected to the first inner surface part 41A, the first outer surface part 41B, and the first side surface part 41C of the shaft-like part 41, respectively. The shaft-like part 41 and the finger hook part 42 extend toward the Z(+) direction side with the disk part 48 as a starting point. The shaft-like part 41 and the finger hook part 42 may extend linearly, or the finger hook part 42 may be inclined with respect to the shaft-like part 41. Even in the case of the substantially I-shaped operation lever that does not have the bent part 43, the operator can easily operate the operation lever by having the inclined surface part.

In addition, in the operation lever of another form, the shape of the side surface part of the shaft-like part may be different from the shape of the side surface part of the shaft-like part 41 of the embodiment. Specifically, the shaft-like part of the operation lever of another form does not have the first change region unlike the side surface part of the shaft-like part 41. That is, the width of the shaft-like part in the rotation axis C direction is constant from the rotation axis C side toward the finger hook part 42 side. Even in a case of an operation lever having another form, the operator can easily operate the operation lever by having the inclined surface part on the side surface part.

### Other Preferred Forms

In the embodiment, the ultrasound probe 10 has been described as an example of a target to which an operation lever according to the embodiment of the present invention is applied. However, the present invention is not limited to the ultrasound probe 10 and can be applied to various medical devices. That is, the present invention can be applied to, for example, an endoscope comprising a laparoscope and a soft insertion part, as long as the medical device has an operation lever for executing an operation. In this case, an optical imaging apparatus is provided on a distal end side of the insertion part as an observation window.

Although the operation lever according to the embodiment of the present invention has been described above, the present invention may be improved or modified in some ways without departing from the gist of the present invention.

### Explanation of References

10: ultrasound probe
12: operation part
14: insertion part
16: insertion part main body
18: bendable part
20: probe
20A: ultrasound wave emission surface
22: distal end part
22A: diameter-reduced part
24: operation part main body
26: grip part
26A: proximal end part
28: cable
30: operation lever
30A: inner surface part
30B: outer surface part
30C: side surface part
30E: first inclined surface part
30F: second inclined surface part
30G: third inclined surface part
30H: fourth inclined surface part
32: operation lever
41: shaft-like part
41A: first inner surface part
41B: first outer surface part
41B1: first off-axis region
41B2: second off-axis region
41B3: third off-axis region
41C: first side surface part
41C1: first axis side region
41C2: second axis side region
41C3: third axis side region
42: finger hook part
42A: second inner surface part
42B: second outer surface part
42C: second side surface part
43: bent part
43A: third inner surface part
43B: third outer surface part
43C: third side surface part
48: disk part
50: holding surface
52: holding surface
54: arc surface
56: arc surface
60: inclined surface
62: inclined surface
100: left hand
102: thumb
103: index finger
104: middle finger
105: ring finger
106: little finger
A: longitudinal axis
C: rotation axis
WA1: width
WA2: width
WA3: width
WB1: width
WB2: width
WB3: width
WB4: width
WB5: width
WC1: width
WC2: width
WC3: width
α1: inclined angle
α2: inclined angle
α3: inclined angle

## Claims

1. An operation lever that is attached to an operation part for operating an insertion part to be inserted into a subject so as to be rotatable about a rotation axis, the operation lever comprising:
an inner surface part that faces a side of the operation part;
an outer surface part that faces a side opposite to the side of the operation part and has a width in a longitudinal direction of the operation part smaller than a width of the inner surface part; and
an inclined surface part that is provided between the outer surface part and the inner surface part and is inclined with respect to the outer surface part.

2. The operation lever according to claim 1,
wherein the inclined surface parts are provided on both sides of the outer surface part in the longitudinal direction, respectively.

3. The operation lever according to claim 1 or 2,
wherein the inclined surface part is connected to the outer surface part.

4. The operation lever according to any one of claims 1 to 3, further comprising:
a finger hook part that is provided at a position away from the rotation axis and extends in a direction in which the rotation axis extends; and
a shaft-like part that is provided between the rotation axis and the finger hook part and extends in a direction intersecting the rotation axis,
wherein the inclined surface part is provided on the finger hook part or the shaft-like part.

5. The operation lever according to claim 4, further comprising:
a bent part that is provided between the finger hook part and the shaft-like part.

6. The operation lever according to claim 4 or 5,
wherein the shaft-like part has a side surface part having a normal component in the longitudinal direction, and
the inclined surface part is provided on the side surface part.

7. The operation lever according to claim 6,
wherein the side surface part includes
a first region provided on a side of the rotation axis,
a second region provided on a side of the finger hook part with respect to the first region and having a width in a direction of the rotation axis larger than a width of the first region, and
a first change region provided between the first region and the second region and having a width in the direction of the rotation axis larger on a side of the second region than on a side of the first region, and
the inclined surface part is provided on the second region or the first change region.

8. The operation lever according to any one of claims 4 to 7,
wherein the outer surface part is provided on the shaft-like part, and
the outer surface part of the shaft-like part includes
a third region provided on a side of the rotation axis,
a fourth region provided on a side of the finger hook part with respect to the third region and having a width in the longitudinal direction smaller than a width of the third region, and
a second change region provided between the third region and the fourth region and having a width in the longitudinal direction smaller on a side of the fourth region than on a side of the third region.

9. The operation lever according to any one of claims 4 to 8,
wherein the inclined surface part is provided on the finger hook part.

10. A medical device including the insertion part and the operation part, comprising:
at least one operation lever according to any one of claims 1 to 9.

11. The medical device according to claim 10,
wherein the at least one operation lever includes a first operation lever and a second operation lever, and
the first operation lever and the second operation lever are disposed to face the operation part.

12. The medical device according to claim 11,
wherein the first operation lever and the second operation lever bend the insertion part in different directions.

13. An ultrasound probe including the insertion part and the operation part and having a probe provided on a distal end side of the insertion part, the ultrasound probe comprising:
at least one operation lever according to any one of claims 1 to 9.

14. An endoscope including the insertion part and the operation part and having an observation window provided on a distal end side of the operation part, the endoscope comprising:
at least one operation lever according to any one of claims 1 to 9.
